Europäisches Patentamt

European Patent Office

Office européen des brevets

(11) Veröffentlichungsnummer: **0 058 744**
**B1**

# (12) EUROPÄISCHE PATENTSCHRIFT

(45) Veröffentlichungstag der Patentschrift:
09.05.84

(51) Int. Cl.³: **A 61 F 1/03**

(21) Anmeldenummer: 81106820.4

(22) Anmeldetag: 01.09.81

(54) Spreizbare Markraumsperre.

(30) Priorität: 18.02.81 CH 1064/81

(43) Veröffentlichungstag der Anmeldung:
01.09.82 Patentblatt 82/35

(45) Bekanntmachung des Hinweises auf die Patenterteilung:
09.05.84 Patentblatt 84/19

(84) Benannte Vertragsstaaten:
AT DE FR GB IT NL

(56) Entgegenhaltungen:
EP - A - 0 023 787
DE - A - 2 063 650
GB - A - 2 017 503
US - A - 4 275 717

(73) Patentinhaber: **GEBRÜDER SULZER AKTIENGESELLSCHAFT**, Zürcherstrasse 9, CH-8401 Winterthur (CH)

(72) Erfinder: **Weber, Bernhard Georg, Prof. Dr.-med.**, Kantonsspital, CH-9000 St. Gallen (CH)

(74) Vertreter: **Dipl.-Ing. H. Marsch Dipl.-Ing. K. Sparing Dipl.-Phys.Dr. W.H. Röhl Patentanwälte**, Rethelstrasse 123, D-4000 Düsseldorf (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine spreizbare Markraumsperre zum Einsetzen in den sich erweiternden Markraum von Röhrenknochen, in die durch die Operationsöffnung Verankerungsschäfte von Endoprothesen eingesetzt und dort mit Hilfe von Knochenzement verankert werden.

Für den inneren Abschluss des Markraumes von Röhrenknochen, in die Verankerungsschäfte von Endoprothesen eingesetzt werden, sind bereits Einsätze bekannt, die aus einem Kern und elastischen Lappen bestehen, mit denen sich die Einsätze in der Markhöhle verankern; die elastischen Lappen schieben sich dabei blüttenblattartig übereinander und bilden einen pfropfenartigen Abschluss für den zur Verankerung vorgesehenen Markraumabschnitt, so dass der eingefüllte Knochenzement beim Eintreiben des Prothesenschaftes nicht axial in den Markraum abfliessen kann, sondern möglichst weitgehend gegen die Seitenwände der Markhöhle verdrängt wird (DE-C- 28 14 037).

Voraussetzung für ein widerstandsfähiges Haften der bekannten Einsätze an den Seitenwänden des Röhrenknochens ist, dass der Markraum des Knochens sich verengt oder zumindest sich nicht erweitert. Diese Voraussetzung ist nicht in allen Fällen gegeben. So ist der Markraum des Femurs sanduhrförmig: Er erweitert sich von der Femurmitte nach oben und auch gegen das distale Ende des Femurknochens. Werden am proximalen Ende des Femurknochens Femurkopf-Prothesen mit überlangen Verankerungsschäften eingesetzt, so ist es also möglich, dass der pfropfenartige Einsatz, vom proximalen Ende des Knochens her gesehen, jenseits der den engsten Hohlraumquerschnitt aufweisenden Mitte des Knochens in den erwähnten, sich erweiternden Bereich des Markhohlraumes von der proximalen Seite her eingesetzt werden muss.

Aufgabe der Erfindung ist, für derartige, sich nach «innen» erweiternde Markraumkanäle eine geeignete Markraumsperre zu schaffen; diese Aufgabe wird erfindungsgemäss dadurch gelöst, dass durch einen haubenartigen Aussenkörper, dessen Mantel durch Unterteilen in mindestens zwei aussen mit widerhakenartig wirkenden Verankerungselementen versehene Segmente aufspreizbar ist, und ferner durch einen konischen Spreizkörper, der in den Innenhohlraum des haubenartigen Aussenkörpers einziehbar und in diesem fixierbar ist.

Bei der neuen Konstruktion können der vorteilhafterweise elastische Spreizkörper und der geschlossenen haubenartige Aussenkörper nacheinander – aber mit Vorteil gemeinsam auf ein Instrument aufgeschraubt – durch die relativ enge Operationsöffnung in den Knochen eingebracht und in dem sich erweiternden Hohlraum durch Einziehen des Spreizkörpers in den Innenhohlraum des Aussenkörpers fixiert werden.

Für die Fixierung des Spreizkörpers im schirmartig aufgespreizten Aussenkörper ist es dabei zweckmässig, wenn die Innenwandfläche des Aussenkörpermantels und die Mantelfläche des Spreizkörpers mit einander entgegengerichteten Verzahnungen versehen sind, die je aus mindestens einem Zahn bestehen. Werden auf mindestens einer der beiden Flächen in axialer Richtung fortschreitende Zahnreihen hintereinander vorgesehen, so lässt sich ein stufenweises Aufspreizen des Aussenkörpers erreichen.

Das Auffädeln der beiden Einzelkörper und das Hineinziehen des Spreizkörpers in den haubenartigen Aussenkörper lassen sich vereinfachen, wenn der Boden des Aussenkörpers und der Spreizkörper zentrale, koaxiale Bohrungen aufweisen, und wenn in der Bohrung des Spreizkörpers Mittel zur lösbaren Befestigung eines Zuginstruments – für den Durchtritt bzw. die Befestigung eines geeigneten Instrumentes – vorgesehen sind.

Im folgenden wird die Erfindung anhand eines Ausführungsbeispiels im Zusammenhang mit der Zeichnung näher erläutert:

Fig. 1 zeigt einen axialen Längsschnitt eines haubenartigen Aussenkörpers im geschlossenen Zustand;

Fig. 2 ist, ebenfalls im axialen Längsschnitt, ein Spreizkörper;

Fig. 3 stellt die beiden Einzelkörper aufgefädelt auf ein geeignetes Einführ- und Zuginstrument dar;

Fig. 4 ist ein Längsschnitt durch einen Röhrenknochen, der in Einsetzrichtung eines Prothesenschaftes (Pfeil A) einen sich erweiternden Markhohlraum aufweist, in dem die neuartige Markraumsperre fixiert ist;

Fig. 5 schliesslich gibt eine Aufsicht auf Fig. 4 in Richtung des Pfeiles A wieder, wobei der die Markraumsperre umgebende Knochen weggelassen worden ist.

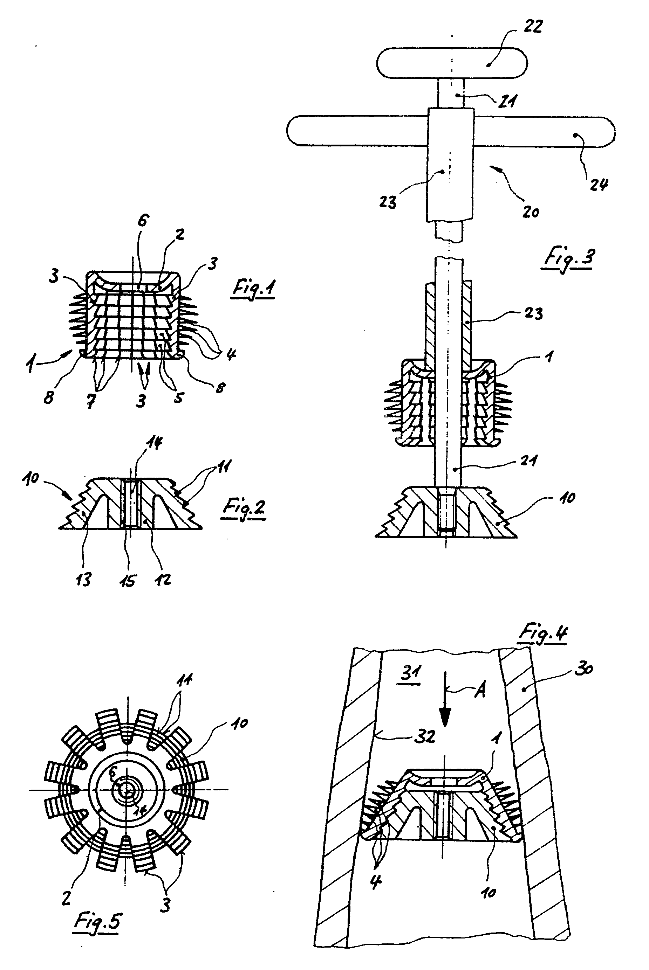

Der aufspreizbare Aussenkörper 1 (Fig. 1) hat im geschlossenen Zustand die Form eines umgedrehten Bechers; sein leicht eingedrückter Boden ist relativ leicht, plastisch oder elastisch, kalt verformbar. Die Wand des Bechers besteht aus einer Anzahl Segmente 3, die durch bis in den Bereich des Bodens 2 verlaufende Trennschnitte 7 durch seinen Mantel entstehen. Die äussere Mantelfläche weist über die Höhe verteilt mehrere Reihen von widerhakenartigen Verankerungselementen 4 auf, die beispielsweise ursprünglich – d.h. bei ungetrenntem Mantel – als Zirkularrippen ausgebildet sind, die ebenfalls durch die Trennschnitte 7 unterteilt werden.

Der freie Rand des «Bechers» endet in einem – für ein erleichtertes Einführen in den Knochen bzw. ein erleichtertes Einziehen des Spreizkörpers 10 – nach aussen abgerundeten, ebenfalls einen Widerhaken bildenden Ansatz 8. Die innere Mantelfläche des «Bechers» ist mit einer Verzahnung 5 versehen; der Boden 2 weist eine zentrale Bohrung 6 auf.

Der eigentliche Spreizkörper 10 besitzt eine konische Grundform, deren äussere Mantelfläche ebenfalls eine Verzahnung 11 trägt, die beim Einziehen des Spreizkörpers 10 in den Aussenkörper 1 im Zusammenwirken mit der Verzahnung 5 beide Einzelkörper unlösbar miteinander verbindet. Der Spreizkörper 10 kann im Querschnitt aus einem Stamm 12 und einem Schirm 13 bestehen, so dass er beim Einführen in den Knochen durch die relativ enge Operationsöffnung unter Umständen elastisch etwa auf die Grösse seines kleineren Durchmessers zusammengepresst werden kann; er trägt ebenfalls eine zentrale Bohrung 14, in die ein Gewinde 15 eingeschnitten ist.

Dieses dient zum Einschrauben eines Einführ- und Zuginstruments 20 (Fig. 3), das am Ende einer Spindel 21 ein entsprechendes Gegengewinde hat. Das Instrument, dessen Länge an die maximale Tiefe, in die eine Markraumsperre gemäss der Erfindung in den Knochen eingeführt werden muss, abgestimmt ist, endet an ihrem anderen Ende in einem Handgriff 22; auf die Spindel 21 wird der Aussenkörper 1 aufgefädelt, ehe der Spreizkörper 10 angeschraubt wird, wobei die Bohrung 6 so an die Spindeldicke angepasst ist, dass der geschlossene Aussenkörper 1 – gegebenenfalls bei gleichzeitiger leichter Abstützung auf dem angeschraubten Spreizkörper 10 – zum Einführen in den Knochen auf der Spindel 21 durch Reibung haftet.

Die Spindel 21 ist von einem, relativ zu ihr axial, verschiebbaren Rohr 23 umgeben, gegen dessen unteres Ende sich der Boden 2 des Aussenkörpers 1 abstützen kann; sein oberes Ende ist mit einem zweiten grösseren Handgriff 24 versehen.

Das Einführen und Fixieren der Markraumsperre in einem Knochen 30 sei nunmehr in Verbindung mit Fig. 4 erläutert. Der dort gezeigte Ausschnitt des Knochens 30 stellt z.B. schematisch einen, vom Femurkopf gesehen, jenseits der Mitte liegenden Abschnitt des Femurknochens dar; im Femur liegt bekanntlich die engste Stelle des Markhohlraumes etwa in der Mitte, von der aus sich der Hohlraum zu den Kondylen hin wieder erweitert. Für den Einsatz eines sehr langen Schaftes einer Hüftgelenkprothese, wie er beispielsweise bei Reoperationsprothesen notwendig ist, ist im Markraum des Knochens operativ eine sich in Richtung des Pfeiles A erweiternde Ausnehmung 31 geschaffen worden, die durch die neuartige Markraumsperre gegen die sich nach unten anschliessenden nicht dargestellten Kondylen hin abgeschlossen werden soll.

Die beiden Einzelkörper der Markraumsperre werden zur Montage im Knochen 30 z.B. in der in Fig. 3 gezeigten Weise am Instrument 20 befestigt und – unter Umständen nach einem Zusammendrücken des elastischen Spreizkörpers 10 – durch die nicht dargestellte, relativ enge Operationsöffnung von oben her in die Ausnehmung 31 eingeführt; die Stellung, bei der sich der Aussenkörper 1 dabei in der für die Lage der Markraumsperre vorgesehene Stelle der Ausnehmung 31 befindet, kann am Rohr 23 durch eine Markierung der dafür notwendigen Tiefe unterhalb der Operationsöffnung angezeigt werden. Ist die richtige Lage für den Aussenkörper 1 erreicht, so wird die Spindel 21 mit Hilfe des Handgriffes 22 relativ zum Rohr 23 nach oben gezogen, wodurch der Spreizkörper 10 in den zuvor geschlossenen Aussenkörper 1 hineingleitet und diesen aufspreizt, da der Aussenkörper 1 durch ein Anliegen seines Bodens 2 am unteren Ende des Rohres 23 an einem Ausweichen nach oben gehindert ist. Der in die Haube des Aussenkörpers 1 eindringende Spreizkörper 10 drückt dabei die einzelnen Segmente 3 des Aussenkörpers 1 allseitig auseinander, wobei sich die Verankerungselemente 4 in Unebenheiten an der Innenwand 32 des kortikalen Knochengewebes verhaken. Gleichzeitig verschliesst der Spreizkörper 10 die beim Aufspreizen zwischen den geöffneten Segmenten 3 entstandenen Zwischenräume, wie aus Fig. 5 zu entnehmen ist.

Eine unlösbare Verbindung zwischen dem aufgespreizten Aussenkörper 1 und dem Spreizkörper 10 wird dadurch erreicht, dass die beiden Verzahnungen 5 und 11 ineinander greifen.

Es sei noch erwähnt, dass es sich bei der Konstruktion des Instrumentes 20 und der beschriebenen Art der Montage der Markraumsperre nur um ein mögliches Ausführungsbeispiel handelt, durch das die Erfindung in keiner Weise beschränkt wird.

Als bevorzugtes Material für die beiden Einzelkörper 1 und 10 dienen die in üblicherweise in der Implantattechnik verwendeten körperverträglichen und körperbeständigen Kunststoffe, insbesondere Polyäthylen der Klassifikation HDPE und UHMW. Es ist jedoch auch möglich, die beiden Einzelkörper aus einem der für Endoprothesen üblichen Metalle oder Metall-Legierungen zu fertigen.

Um Markräume mit stark unterschiedlichen Durchmessern verschliessen zu können, ist es zweckmässig, im Rahmen einer Typenreihe Aussenkörper und Spreizkörper unterschiedlichen Durchmessers bereit zu halten. So lassen sich beispielsweise mit drei verschiedenen Grössen Markraumdurchmesser von 10 bis 35 mm absperren.

**Patentansprüche**

1. Spreizbare Markraumsperre zum Einsetzen in den sich erweiternden Markraum von Röhrenknochen in die durch die Operationsöffnung Verankerungsschäfte von Endoprothesen eingesetzt und dort mit Hilfe von Knochenzement verankert werden, gekennzeichnet durch einen haubenartigen Aussenkörper (1), dessen Mantel durch Unterteilen in mindestens zwei aussen mit widerhakenartig wirkenden Verankerungselementen (4) versehene Segmente (3) aufspreizbar ist, und ferner durch einen konischen Spreizkörper (10), der in den Innenhohlraum des haubenartigen Aussenkörpers (1) einziehbar und in diesem fixierbar ist.

2. Markraumsperre nach Anspruch 1, dadurch gekennzeichnet, dass die Innenwandfläche des Aussenkörpermantels und die Mantelfläche des Spreizkörpers mit einander entgegengerichteten Verzahnungen (5 und 11) versehen sind.

3. Markraumsperre nach Anspruch 1, dadurch gekennzeichnet, dass der konische Spreizkörper (10) elastisch verformbar ist.

4. Markraumsperre nach Anspruch 1, dadurch gekennzeichnet, dass der Boden (2) des Aussenkörpers (1) und der Spreizkörper (10) zentrale, koaxiale Bohrungen (6 und 14) aufweisen.

5. Markraumsperre nach Anspruch 4, dadurch gekennzeichnet, dass in der Bohrung (14) des Spreizkörpers (10) Mittel (15) zur lösbaren Befestigung eines Zuginstrumentes (20) vorgesehen sind.

## Claims

1. An expandable medullary space plug for insertion into the widening medullary space of medullated bones, into which anchoring stems of endoprostheses are inserted through the operation opening and are anchored therein by means of bone cement, characterised by an outer member (1) in the form of a hood, the surface of which is expandable by sub-division into at least two segments (3) having on the outside anchoring elements (4) acting in the form of barbs, and also by a conical expansion member (10) which is adapted to be pulled into and secured in the inner cavity of the outer member (1).

2. A medullary space plug according to claim 1, characterised in that the inner wall surface of the outer member and the outer surface of the expansion member are provided with teeth (5 and 11) extending in opposite directions.

3. A medullary space plug according to claim 1, characterised in that the conical expansion member (10) is elastically deformable.

4. A medullary space plug according to claim 1, characterised in that the base (2) of the outer member (1) and the expansion member (10) have central coaxial bores (6 and 14).

5. A medullary space plug according to claim 4, characterised in that means (15) for releasably securing a traction instrument (20) are provided in the bore (14) of the expansion member (10).

## Revendications

1. Marqueur dilatable destiné à être introduit dans l'espace de marquage qui s'élargit d'os tubulaires dans lesquels des tiges d'ancrage d'endoprothèses sont insérées par l'ouverture d'opération et sont ancrées à cet endroit à l'aide de ciment pour os, caractérisé par un corps externe (1) du type calotte dont l'enveloppe peut se dilater par sa subdivision en au moins deux segments (3) munis à l'extérieur d'éléments d'ancrage (4) agissant à la manière des barbelés, ainsi que par un corps conique de dilatation (10) qui peut être introduit et fixé dans l'espace interne creux du corps externe (1) du type calotte.

2. Bloqueur selon la revendication 1, caractérisé en ce que la face de paroi interne de l'enveloppe du corps externe et la face enveloppe du corps de dilatation sont munies de dentures (5 et 11) de sens opposés.

3. Bloqueur selon la revendication 1, caractérisé en ce que le corps de dilatation conique (10) peut être déformé élastiquement.

4. Bloqueur selon la revendication 1, caractérisé en ce que le fond (2) du corps externe (1) et le corps de dilatation (10) présentent des alésages coaxiaux centraux (6 et 14).

5. Bloqueur selon la revendication 4, caractérisé en ce qu'on prévoit dans l'alésage (14) du corps de dilatation (10) des moyens (15) permettant une fixation amovible d'un instrument de traction (20).

Fig.1

Fig.2

Fig.3

Fig.4

Fig.5